# FASCICULE DE BREVET EUROPEEN

(11) **EP 2 097 516 B1**
(45) Date de publication et mention de la délivrance du brevet: **14.11.2012**
(21) Numéro de dépôt: 07872388.9
(22) Date de dépôt: 18.12.2007
(51) Int. Cl.: C12N 7/01

(54) **PROCÉDÉ DE PRÉPARATION DE BACTÉRIOPHAGES MODIFIÉS PAR INSERTION DE SÉQUENCES ALÉATOIRES DANS LES PROTÉINES DE CIBLAGE DESDITS BACTÉRIOPHAGES**
VERFAHREN FÜR DIE HERSTELLUNG VON MITTELS EINFÜGUNG ZUFÄLLIGER SEQUENZEN IN DIE SCREENING-PROTEINE MODIFIZIERTEN BAKTERIOPHAGEN
METHOD FOR PREPARING BACTERIOPHAGES MODIFIED BY THE INSERTION OF RANDOM SEQUENCES IN THE SCREENING PROTEINS OF SAID BACTERIOPHAGES

(30) Priorité: 20.12.2006 FR 0655721
(43) Date de publication de la demande: 09.09.2009
(73) Titulaire: Pherecydes Pharma, 93230 Romainville (FR)
(72) Inventeur: IRIS, François, F-92370 Chaville (FR)
(74) Mandataire: Caen, Thierry Alain
(86) Numéro de dépôt international: PCT/FR2007/002101
(87) Numéro de publication internationale: WO 2008/093009

(56) Documents cités:
- EP-A- 0 698 091
- WO-A-2006/066224
- US-A1- 2003 216 338
- BREDO ET AL: "Gene reconstitution using high efficiency homologous recombination between a bacteriophage fd and a plasmid" JOURNAL OF BIOTECHNOLOGY, ELSEVIER SCIENCE PUBLISHERS, AMSTERDAM, NL, vol. 126, no. 3, 10 novembre 2006 (2006-11-10), pages 286-290, XP005703077 ISSN: 0168-1656
- YOICHI M ET AL: "Alteration of tail fiber protein gp38 enables T2 phage to infect Escherichia coli O157:H7" JOURNAL OF BIOTECHNOLOGY, ELSEVIER SCIENCE PUBLISHERS, AMSTERDAM, NL, vol. 115, no. 1, 12 janvier 2005 (2005-01-12), pages 101-107, XP004966993 ISSN: 0168-1656
- SWITALA-JELEN K ET AL: "Mutations in bacteriophage T4 genome" ACTA VIROLOGICA, ACADEMIA PRAGUE, PRAGUE, CS, vol. 46, no. 2, 2002, pages 57-62, XP008025059 ISSN: 0001-723X
- DESPLATS CARINE ET AL: "The diversity and evolution of the T4-type bacteriophages." RESEARCH IN MICROBIOLOGY, vol. 154, no. 4, mai 2003 (2003-05), pages 259-267, XP002445692 ISSN: 0923-2508
- TETART F ET AL: "Genome plasticity in the distal tail fiber locus of the T-even bacteriophage: recombination between conserved motifs swaps adhesin specificity" JOURNAL OF MOLECULAR BIOLOGY, LONDON, GB, vol. 282, no. 3, 25 septembre 1998 (1998-09-25), pages 543-556, XP004455941 ISSN: 0022-2836

### LISTAGE DE SEQUENCES

<110> PHERECYDES PHARMA
<120> Procédé de préparation de bactériophages modifiés par insertion de séquences aléatoires dans les protéines de ciblage desdits bactériophages
<130> BIF 117138 WO
<160> 13
<170> PatentIn version 3.1
<210> 1
   <211> 1582
   <212> DNA
   <213> bacteriophage T4
<220>
   <221> misc_feature
   <222> (1)..(1582)
   <223> Région codante du gène gp12 de bactériophage T4
<400> 1
   gagtaataat acatatcaac acgtttctaa tgaatctcgt tatgtaaaat ttgatcctac   60
   cgatacgaat tttccaccgg agattactga tgttcacgct gctatagcag ccatttctcc  120
   tgctggagta aatggagttc ctgatgcatc gtcaacaaca aagggaattc tatttattcc  180
   cactgaacag gaagttatag atggaactaa taataccaaa gcagttacac cagcaacgtt  240
   ggcaacaaga ttatcttatc caaatgcaac tgaaactgtt tacggattaa caagatattc  300
   aaccaatgat gaagccattg ccggagttaa taatgaatct tctataactc cagctaaatt  360
   tactgtcgcc cttaataatg cgtttgaaac gcgagtttca actgaatcct caaatggtgt  420
   tattaaaatt tcatctctac cgcaagcatt agctggtgca gatgatacta ctgcaatgac  480
   tccattaaaa acacagcagt tagctattaa attaattgcg caaattgctc cttctgaaac  540
   cacagctacc gaatcggacc aaggtgttgt tcaattagca acagtagcgc aggttcgtca  600
   gggaacttta agagaaggct atgcaatttc tccttatacg tttatgaatt catcttctac  660
   tgaagaatat aaaggcgtaa ttaaattagg aacacaatca gaagttaact cgaataatgc  720
   ttctgttgcg gttactggcg caactcttaa tggtcgtggt tctacgacgt caatgagagg  780
   cgtagttaaa ttaactacaa ccgccggttc acagagtgga ggcgatgctt catcagcctt  840
   agcttggaat gctgacgtta tccagcaaag aggtggtcaa attatctatg gaacactccg  900
   cattgaagac acatttacaa tagctaatgg tggagcaaat attacgggta ccgtcagaat  960
   gactggcggt tatattcaag gtaaccgcat cgtaacacaa aatgaaattg atagaactat 1020
   tcctgtcgga gctattatga tgtgggccgc tgatagtctt cctagtgatg cttggcgctt 1080
   ctgccatggt ggaactgttt cagcgtcaga ttgtccatta tatgcttcta gaattggaac 1140
   aagatatggc ggaaacccat caaatcctgg attgcctgac atgcgtggtc tttttgttcg 1200
   tggttctggt cgtggttctc acttaacaaa tccaaatgtt aatggtaatg accaatttgg 1260
   taaacctaga ttaggtgtag gttgtaccgg tggatatgtt ggtgaagtac agatacaaca 1320
   gatgtcttat cataaacatg ctggtggatt tggtgagcat gatgatctgg gggcattcgg 1380
   taatacccgt agatcaaatt ttgttggtac acgtaaagga cttgactggg ataaccgttc 1440
   atacttcacc aatgacggat atgaaattga cccagaatca caacgaaatt ccaaatatac 1500
   attaaatcgt cctgaattaa ttggaaatga aacacgtcca tggaacattt ctttaaacta 1560
   cataattaag gtaaaagaat ga 1582
<210> 2
   <211> 24
   <212> DNA
   <213> Séquence artificielle
<220>
   <221> Amorce
   <222> (1)..(24)
   <223> Amorce gp12F pour l'amplification du gène gp12 du bacteriophage T4
<400> 2
   tgagtaataa tacatatcaa cacg 24
<210> 3
   <211> 25
   <212> DNA
   <213> Séquence artificielle
<220>
   <221> Amorce
   <222> (1)..(25)
   <223> Amorce gp12R pour l'amplification du gène gp12 du bacteriophage T4
<400> 3
   tgattctttt accttaatta tgtac 25
<210> 4
   <211> 21
   <212> DNA
   <213> Séquence artificielle
<220>
   <221> Amorce
   <222> (1)..(21)
   <223> Amorce P12NF1F interne du domaine d'ancrage de gp12 du
   bacteriophage T4
<400> 4
   tcaaggtaac cgcatcgtaa c 21
<210> 5
   <211> 18
   <212> DNA
   <213> Séquence artificielle
<220>
   <221> Amorce
   <222> (1)..(18)
   <223> Amorce P12NF2R interne du domaine d'ancrage de gp12 du
   bacteriophage T4
<400> 5
   aaagaccacg catgtcag 18
<210> 6
   <211> 20
   <212> DNA
   <213> Séquence artificielle
<220>
   <221> Amorce
   <222> (1)..(20)
   <223> Amorce P12NF2F interne du domaine d'ancrage de gp12 du
   bacteriophage T4
<400> 6
   tgccatggtg gaactgttca 20
<210> 7
   <211> 18
   <212> DNA
   <213> Séquence artificielle
<220>
   <221> Amorce
   <222> (1)..(18)
   <223> Amorce P12NF3R interne du domaine d'ancrage de gp12 du
   bacteriophage T4
<400> 7
   cacctaatct acgtttac 18
<210> 8
   <211> 18
   <212> DNA
   <213> Séquence artificielle
<220>
   <221> Amorce
   <222> (1)..(18)
   <223> Amorce P12NF3F interne du domaine d'ancrage de gp12 du
   bacteriophage T4
<400> 8
   ctgacatgcg tggtcttt 18
<210> 9
   <211> 18
   <212> DNA
   <213> Séquence artificielle
<220>
   <221> Amorce
   <222> (1)..(18)
   <223> Amorce P12NF4R interne du domaine d'ancrage de gp12 du
   bacteriophage T4
<400> 9
   atgtttatga taagacat 18
<210> 10
   <211> 18
   <212> DNA
   <213> Séquence artificielle
<220>
   <221> Amorce
   <222> (1)..(18)
   <223> Amorce P12NF4F interne du domaine d'ancrage de gp12 du
   bacteriophage T4
<400> 10
   gtaaacctag attaggtg 18
<210> 11
   <211> 21
   <212> DNA
   <213> Séquence artificielle
<220>
   <221> Amorce
   <222> (1)..(21)
   <223> Amorce P12NF5R interne du domaine d'ancrage de gp12 du
   bacteriophage T4
<400> 11
   tcattctttt accttaatta t 21
<210> 12
   <211> 24
   <212> DNA
   <213> Séquence artificielle
<220>
   <221> Amorce
   <222> (1)..(24)
   <223> Amorce gp12F pour l'amplification du gène gp12 du bacteriophage T4
<400> 12
   tgagtaataa tacatatcaa cacg 24
<210> 13
   <211> 21
   <212> DNA
   <213> Séquence artificielle
<220>
   <221> Amorce
   <222> (1)..(21)
   <223> Amorce gp12AR pour l'amplification du gène gp12 du bacteriophage T4
<400> 13
   gttacgatgc ggttaccttg t 21

## Description

Le présent document vise un procédé pour obtenir une diversité de bactériophages recombinants dont les protéines de ciblage ont été modifiées de manière aléatoire, ainsi que des banques de bactériophages pouvant être obtenus par ce procédé.

Les bactériophages se présentent comme des virus capables d'infecter spécifiquement les bactéries et de s'y répliquer. Leur existence a été mise en évidence au début du XX^{eme} siècle par le Britannique Frederick Twort et le Québécois Félix d'Hérelle.

Les bactériophages occupent toutes les niches écologiques où se trouvent des bactéries. Ils se manifestent principalement sous deux formes : la forme lysogénique, par laquelle ils peuvent demeurer de manière quiescente à l'intérieur de leur hôte, ou bien sous forme lytique, lorsqu'ils se répliquent de manière active en lysant la cellule bactérienne. La forme lytique amène les bactériophages à être libéré en grand nombre dans l'environnement sous une forme infectieuse.

Afin de conserver leur caractère infectieux vis-à-vis de leurs hôtes qui connaissent parfois des mutations rapides, les bactériophages sont amenés à évoluer constamment. De ce fait, Ils présentent naturellement un degré élevé de spécialisation pour les espèces bactériennes qu'ils parasitent et sont très diversifiés.

Dès leur découverte, les bactériophages ont été considérés comme un moyen de lutter contre les infections bactériennes, et ce bien avant l'ère des antibiotiques.

La démarche consistant à identifier dans la nature des bactériophages spécifique d'une bactérie pathogène afin de traiter les patients infectés par cette bactérie s'est ainsi développée en Russie et dans les pays de l'ancien bloc soviétique pendant la première moitié du XXeme siècle.

Cependant l'emploi des antibiotiques, dont le spectre est généralement beaucoup plus large, s'est généralisé dans la seconde moitié du XXeme siècle de façon massive, sans exploiter toutes les possibilités offertes par les bactériophages.

Aujourd'hui, face à l'apparition de souches bactériennes multi-résistantes aux antibiotiques, et au vu des difficultés rencontrées pas la communauté scientifique pour mettre au point de nouveaux antibiotiques, les bactériophages suscitent un nouvel intérêt pour le traitement des infections bactériennes difficiles à éradiquer, notamment en cas de contaminations nosocomiales [Thiel, K., Nature Biotechnology, 2004, 22 :31-36].

Certaines difficultés persistent toutefois dans l'utilisation des bactériophages, notamment dans le fait que les bactéries peuvent échapper aux bactériophages en masquant ou en modifiant les éléments constitutifs de leur paroi externe.

Le cycle de réplication des bactériophages nécessite, en effet, une étape de reconnaissance et d'adhésion du bactériophage à la paroi de la bactérie hôte, laquelle conditionne la possibilité pour le bactériophage d'infecter la bactérie, c'est-à-dire d'injecter le matériel génétique contenu dans sa capside à l'intérieur du cytoplasme de la bactérie.

Le bactériophage T4, par exemple, qui est un bactériophage qui infecte les bactéries de type *Escherichia coli,* a un cycle de réplication qui dure environ 30 minutes à 37 °C. Ce cycle de réplication débute immédiatement après la reconnaissance de la bactérie hôte par le bactériophage, par la phase d'absorption et de pénétration. Il se traduit par l'arrêt immédiat de l'expression des gènes de la bactérie hôte, la synthèse des enzymes nécessaires à la réplication du bactériophage, 5 minutes après l'infection, puis la réplication de l'ADN (démarrant après 10 minutes) et la formation du virus (démarrant après 12 minutes). Le cycle de réplication conduit à l'éclatement de la bactérie (après 30 minutes) et la libération dans l'environnement d'environ cinquante bactériophages par bactérie lysée.

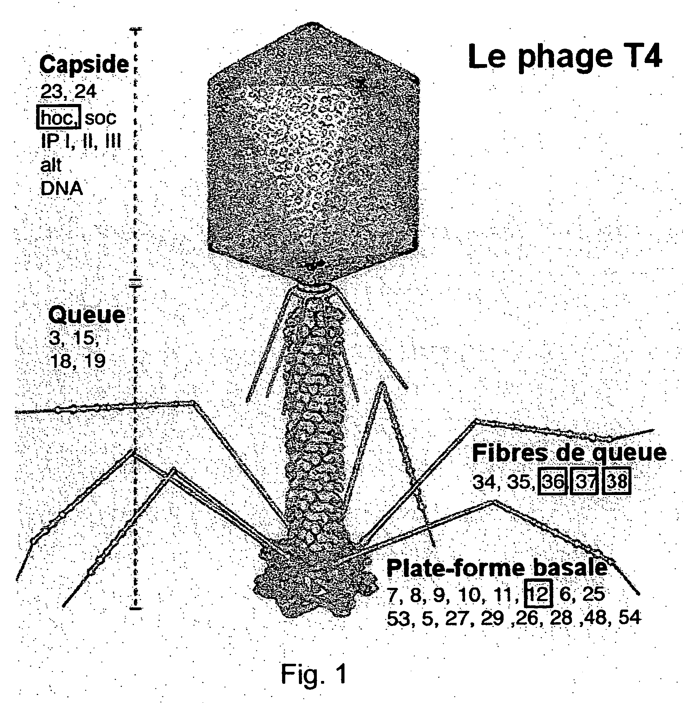

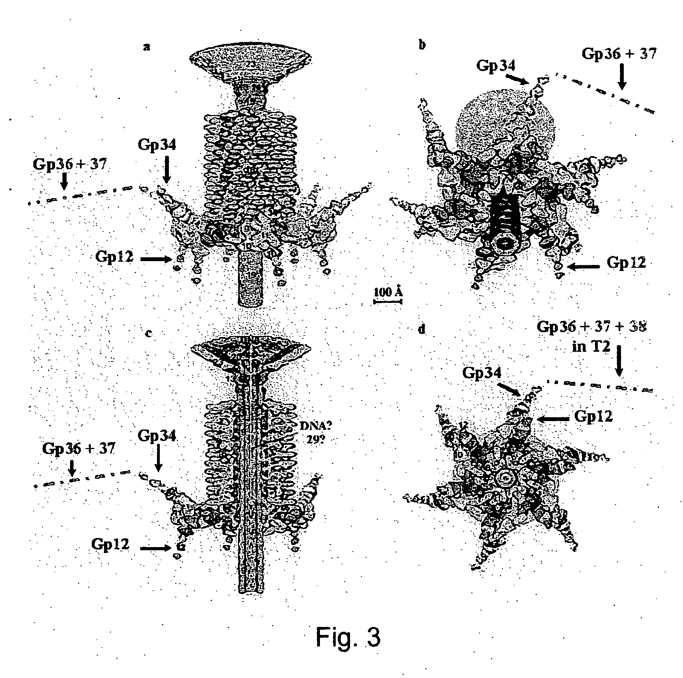

L'adhésion à la bactérie est essentiellement assurée par les protéines de la plate-forme basale (baseplate) servant d'ancrage au bactériophage et la reconnaissance est assurée plus particulièrement par des protéines formant les filaments périphériques, appelées « fibres de queue ». Néanmoins, les protéines de fibres de queue et du baseplate peuvent être à la fois impliquées dans la reconnaissance et l'adhésion du bactériophage à la paroi bactérienne. L'ensemble de ces protéines dites de « ciblage » est représenté sur les figures 1 et 3 de la présente demande.

Parmi les protéines impliquées dans cette reconnaissance ou cette adhésion chez le bactériophage T4, on peut citer plus particulièrement les glycoprotéines GP12 de la plate forme basale, et les glycoprotéines GP36, GP37 et GP38 des fibres de queue.

Afin de limiter l'émergence de bactéries résistantes au dispositif de reconnaissance des bactériophages, il est généralement proposé d'utiliser simultanément différents formes de bactériophages capables de cibler une même bactérie.

Ces bactériophages sont prélevés dans la nature ou issus de collections, pour former ensemble ce qu'on appelle « un cocktail de bactériophages ».

Cependant, il convient pour la mise au point de ces cocktails de bactériophages de sélectionner individuellement et rigoureusement les bactériophages qui les composent, en s'assurant, notamment, que ces bactériophages sont lytiques et non lysogèniques ou partiellement lysogèniques, comme c'est le cas souvent des bactériophages prélevés dans le milieu naturel.

La nécessité de tester individuellement les bactériophages pour s'assurer de leur efficacité réelle rend le développement des cocktails de bactériophages long et fastidieux, et ce d'autant qu'il faut prévoir un cocktail différent pour chaque bactérie considérée.

La demande WO 01/51066 décrit une telle préparation de bactériophages comprenant six bactériophages différents, utilisée en tant qu'agent de conservation de produits alimentaires frais pour détruire la bactérie *Listeria monocytogenes,* responsable de la listériose. Cette préparation naturelle est conditionnée en pulvérisateur pour être pulvérisée sur de la viande ou des produits laitiers. Elle est sans danger pour l'homme, les animaux ou les plantes, car les bactériophages ne peuvent infecter que des bactéries du genre Listeria et non les cellules des organismes pluricellulaires.

Pour remédier aux problèmes posés par la sélection des bactériophages naturels, il est proposé dans la demande WO 06/066224 une méthode pour obtenir des bactériophages dont les protéines de ciblage sont modifiées en vue de cibler spécifiquement un facteur de virulence donné. Les facteurs de virulence sont des molécules décrites comme étant nécessaires à la bactérie pour développer une infection. Ces molécules sont considérées comme des éléments stables, moins susceptibles de varier au cours de l'infection que les éléments de structures externes comme, par exemple, les lipopolysaccharides. La méthode décrite propose de sélectionner une protéine issue d'un bactériophage naturel (par exemple GP37 du bactériophage T4) capable de reconnaître un facteur de virulence décrit dans la littérature (par exemple OmpC d'*E.coli*), de transférer le gène codant cette protéine dans un bactériophage lambda et d'utiliser le bactériophage lambda pour modifier cette protéine. La modification de la protéine consiste à opérer des échanges entre les différents domaines impliqués dans la reconnaissance du facteur de virulence (par exemples les domaines His de GP37). Ces échanges se produisent au moyen de recombinaisons successives entre domaines homologues communs de ces séquences génétiques, ce qui se traduit par réarrangement des différentes séquences constituant le gène, sans insertion d'ADN exogène.

Pour obtenir, par cette méthode, une diversité de l'ordre de 10⁵ variants différents de la même protéine, il est nécessaire d'effectuer un grand nombre de cycles de réplication successifs des bactériophages dans l'hôte bactérien. En effet, ce sont les recombinaisons successives qui permettent d'obtenir les réarrangements entre des différentes régions homologues et donc de générer de la diversité.

Les bactériophages lambda ainsi obtenus sont testés pour leur capacité à adhérer au facteur de virulence ciblé. Cette méthode, qui s'apparente à la technique du « phage display », permet d'isoler différents variants du bactériophage lambda capables de cibler le facteur de virulence, et ainsi de disposer de différentes protéines de ciblage. Les gènes correspondants à ces différentes protéines de ciblage, peuvent, par la suite, être transférés dans des bactériophages infectieux. Ces bactériophages peuvent alors constituer des cocktails de bactériophages actifs vis-à-vis de la bactérie porteuse du facteur de virulence ciblé au départ.

Cette méthode constitue un progrès dans l'obtention de bactériophages diversifiés pour la mise au point de cocktails de bactériophages. Cependant elle ne peut être mise en oeuvre que pour des bactéries pathogènes cultivables, sur lesquelles une identification préalable des facteurs de virulence a pu être effectuée.

En outre, comme les nombreuses autres méthodes décrites dans l'art antérieur reposant sur le même principe, de nombreux cycles de réplication sont nécessaires pour obtenir des événements de recombinaison entre domaines homologues des protéines, en nombre suffisant pour que les bactériophages parviennent à acquérir la capacité d'infecter des hôtes différents de leurs hôtes habituels. Au cours de ce processus, les bactériophages peuvent perdre la capacité d'infecter l'hôte utilisé pour leur réplication. Dès lors, ils se trouvent éliminés de la sélection et échappent à l'expérimentateur.

Il résulte donc une importante perte de diversité des bactériophages modifiés susceptibles d'être obtenus selon ces méthodes.

Ces méthodes ne permettent d'ailleurs, en général, que l'obtention de bactériophages recombinants dont le spectre d'infection est « étendu », c'est-à-dire qui sont capables d'infecter, en plus de leur hôte initial (généralement *E.coli*) d'autres espèces bactériennes, lesquelles sont souvent très proches sur le plan phylogénétique (bactéries à gram négatif du genre *Salmonella* ou *Shigella*) [Masatoshi Y. et al. (2005) Alteration of tail fiber protein GP38 enables T2 phage to infect E.coli O157:H7 Journal of Biotechnology, 115:101-107]. La production de protéines de ciblage modifiées se heurte donc à la contrainte technique selon laquelle l'obtention des bactériophages est dépendante de l'hôte bactérien dans lequel le bactériophage est transformé puis multiplié.

Pour rendre l'emploi des bactériophages plus universel, il serait utile de disposer de bactériophages infectieux vis-à-vis d'un plus grand nombre d'espèces, par exemple, en créant des bactériophages dont le spectre d'infectivité serait modifié indépendamment de la bactérie hôte nécessaire à leur obtention. De tels bactériophages pourraient être utilisés à l'encontre de nouvelles espèces bactériennes, notamment à l'encontre de bactéries pathogènes émergentes ou à l'origine d'infections nosocomiales.

Pour remédier aux difficultés précitées, la présente invention propose un nouveau procédé consistant à diversifier les protéines de ciblage des bactériophages par insertion dans leurs gènes de séquences d'ADN produites de manière aléatoire.

Ce procédé est conçu pour être mis en oeuvre avec un minimum de cycle de réplication dans la bactérie hôte, voir un seul.

Ce procédé permet d'obtenir une grande diversité de bactériophages recombinants sous forme de banques de bactériophages infectieux prêts à l'emploi, utiles pour traiter, par exemple, des bactéries émergentes ou mutantes.

Le procédé selon l'invention est détaillé ci-après, ainsi que les différents objets pouvant en résulter.
**Figure 1** **:** représentation du bactériophage T4 montrant les différentes éléments constitutifs du bactériophage. Les protéines considérées dans le cadre de la présente invention sont encadrées.

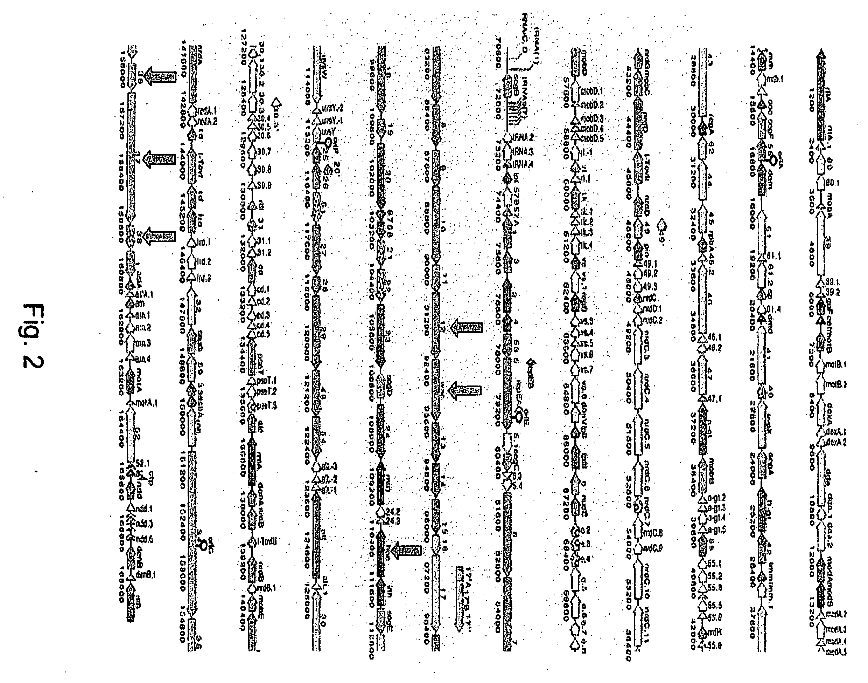

**Figure 2** **:** représentation du génome complet du bactériophage T4. Les gènes cités dans la présente demande sont indiqués à l'aide d'une flèche perpendiculaire aux cadres ouverts de lecture.
**Figure 3** **:** représentation en trois dimensions de la plate-forme basale du bactériophage et des fibres de queue impliquées dans la reconnaissance et l'adhésion du bactériophage à la bactérie hôte, permettant de visualiser les protéines de ciblage modifiées selon le procédé de l'invention.

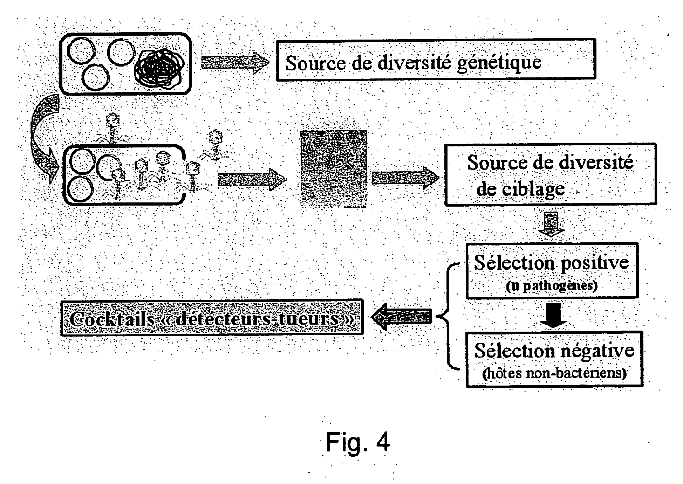

**Figure 4** **:** schéma résumant le principe d'obtention d'une banque de bactériophages recombinants. Le cadre en haut à gauche représente une bactérie hôte comprenant 3 vecteurs de recombinaison homologues visant à introduire des oligonucléotides dont la séquence est produite de manière aléatoire dans trois gènes codant les protéines de ciblage du bactériophage. Ces vecteurs représentent des constructions d'ADN , porteuses d'une grande diversité génétique. Après infection par un bactériophage et recombinaison homologue, un grand nombre de bactériophages (banque de bactériophages) ayant des protéines de ciblage modifiées sont obtenus, formant « une source de diversité de ciblage ». Les bactériophages obtenus sont criblés vis-à-vis de nouveaux hôtes potentiels (sélection positive) afin de sélectionner des bactériophages capables d'infecter ces hôtes. Ils peuvent être également testés sur des hôtes non bactériens (cellules eucaryotes) pour s'assurer qu'ils ne sont pas dangereux pour l'homme ou les animaux.

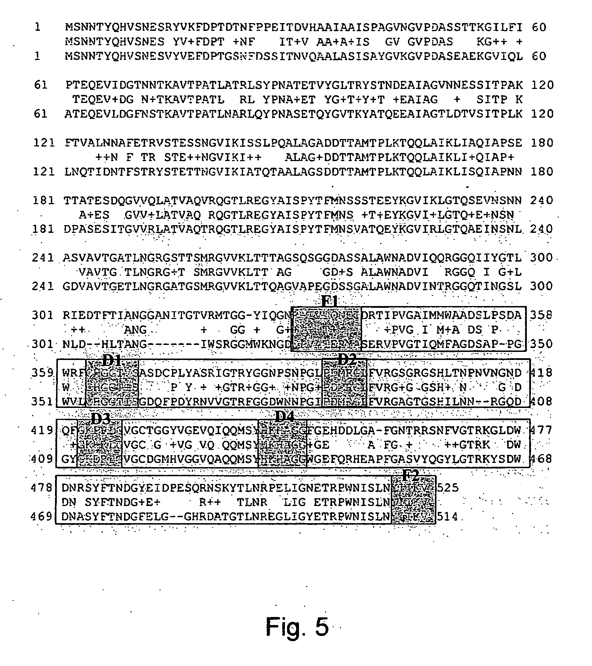

**Figure 5****:** comparaison de la séquence polypeptidique de GP12 du bactériophage T4 (ligne haute) et de son homologue présente dans le bactériophage RB 69 (ligne basse) selon le protocole BLAST. Les acides aminés communs aux deux protéines sont indiqués sur la ligne intermédiaire. Le symbole « + » signifie que les acides aminés sont similaires. La partie N-terminale encadrée correspond au domaine de ces deux protéines qui permet l'ancrage du bactériophage sur la paroi de la bactérie. C'est ce domaine d'ancrage, qui est muté de manière aléatoire puis insérée par recombinaison homologue dans le génome du bactériophage. Les segments internes D1 à D4 correspondent aux séquences de la protéine qui sont conservées au cours du procédé de diversification aléatoire mis en oeuvre par PCR.

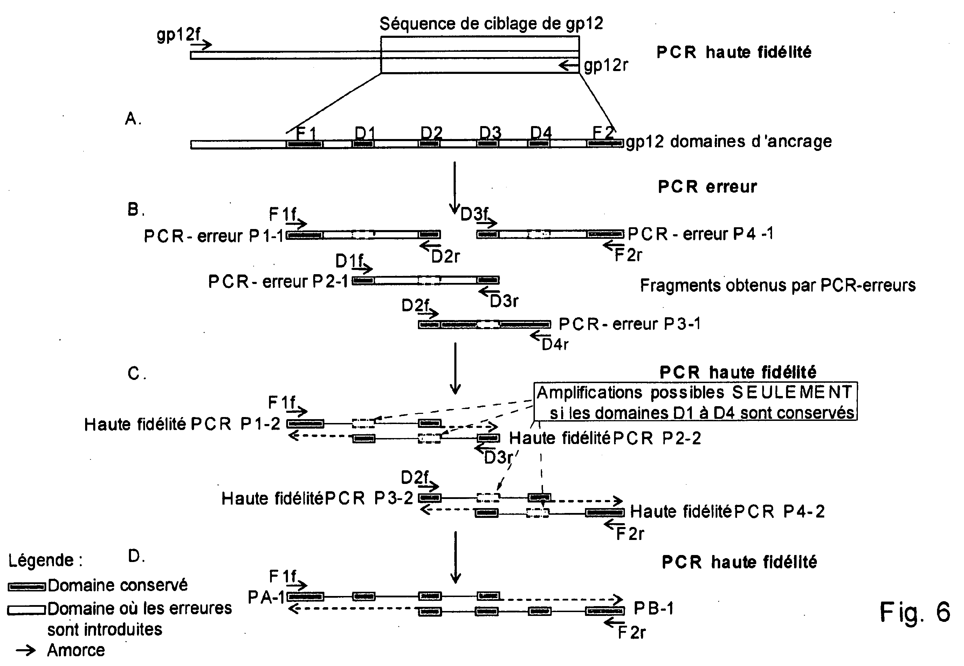

**Figure 6** : récapitulatif des étapes du procédé par PCR mis en oeuvre pour obtenir, notamment, une copie du domaine d'ancrage de gp12 dans laquelle sont insérés des oligonucléotidiques produits de manière aléatoire. Les segments internes D1 à D4 dont on souhaite conserver l'identité de la séquence sont figurés par des rectangles. Ces domaines correspondent à ceux mentionnés dans la figure 5 ci-dessus. Les deux segments externes délimitent la séquence du domaine d'ancrage de gp12. **A:** le domaine d'ancrage de gp12 est amplifié par PCR haute fidélité. **B :** 4 PCR erreur sont réalisées de manière indépendante à l'aide des oligonucléotides mentionnés. C : Les produits d'amplifications obtenus en B sont purifiés et rassemblés dans une même réaction de PCR haute fidélité. Lesdits produits d'amplification se chevauchent si bien qu'il est possible d'assembler les différents fragments, mais à condition que les domaines D1 à D4 soient suffisamment conservés pour pouvoir hybrider les amorces utilisées. **D :** Il résulte de la PCR réalisée à l'étape C, deux fragments PA-1 et PB-1 qui sont assemblés en utilisant les amorces correspondant aux segments externes du domaine d'ancrage de gp12. Au final, on obtient une copie du domaine d'ancrage de gp12 dont la séquence a été modifiée de manière aléatoire sauf au niveau des domaines D1 à D4 dont l'identité a été conservée. Les amorces utilisées dans l'exemple de réalisation ont été reportées à chacune des étapes mentionnées dans cette figure.

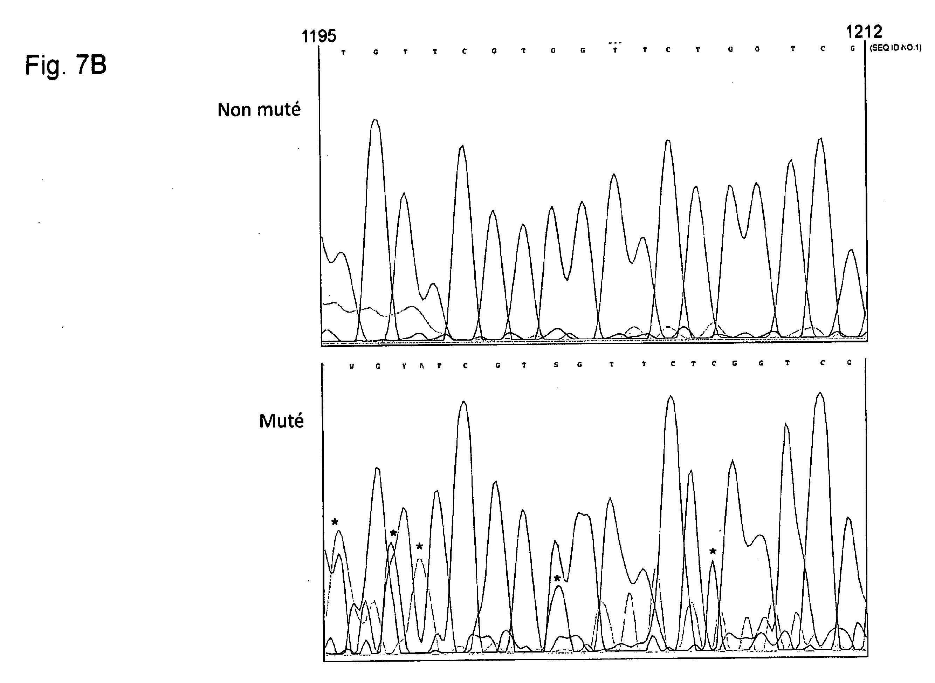

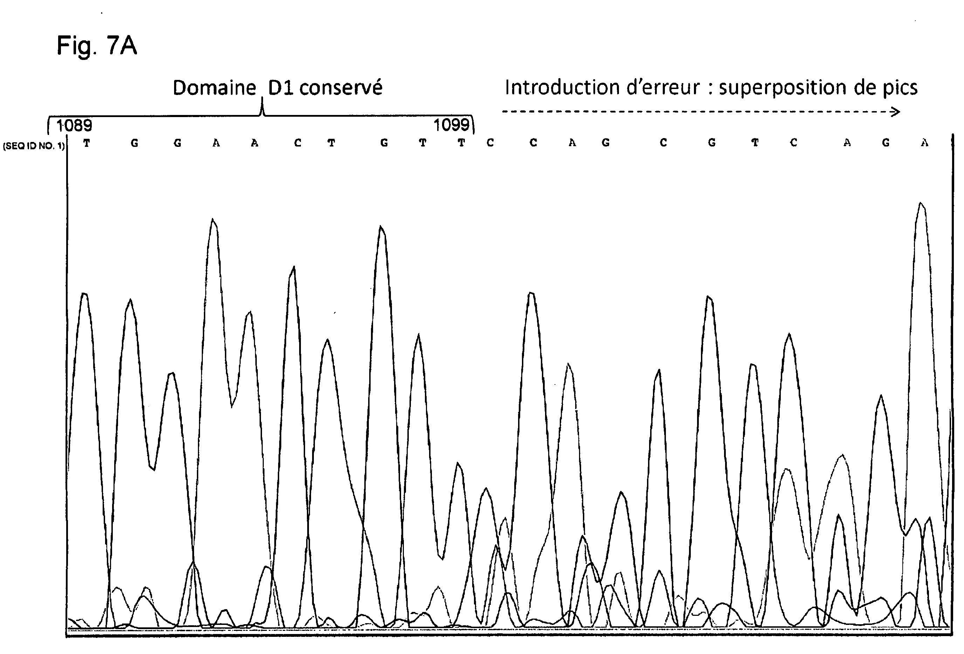

**Figure 7** **:** Profil de séquençage obtenu directement sur les produits de PCR (gp12-Mut) obtenus selon le procédé de l'invention. Les profils ont été établis en utilisant le logiciel d'analyse free ApE à partir des données provenant d'un séquenceur automatique. **A :** séquençage de la portion de gp12 situé entre les nucléotides 1089 et 1110 (SEQ ID NO.1). La partie gauche qui est conservée correspond à la partie 3' du domaine D1 (nucléotides 1089 à 1099). La partie droite montre une superposition de pics rendant compte des mutations aléatoires qui se sont produites au cours du procédé de PCR dans la région située immédiatement en aval de D1 (nucléotides 1100 à 1110). **B:** comparaison du séquençage réalisé dans une région non mutée de gp12 située entre les nucléotides 1195 et 1212 de SEQ ID NO.1 (*en haut*) et réalisé pour la même région sur les produits de PCR obtenus selon le procédé (*en bas*). La région séquencée se trouve entre les domaines conservés D1 et D2. On observe la présence de pics superposés (*en bas*) qui rendent compte de l'insertion aléatoire de nucléotides dans la séquence de *gp12* initiale.

L'invention porte donc sur un procédé de préparation de bactériophages recombinants, lesdits bactériophages prenant la forme d'une banque de bactériophages génétiquement modifiés.

Ce procédé comprend les étapes suivantes :
i) on transforme une bactérie hôte à l'aide d'au moins une construction d'ADN permettant l'insertion d'un oligonucléotide dont la séquence a été produite de manière aléatoire, dans au moins un gène codant une protéine de ciblage d'un bactériophage ;
ii) on infecte les bactéries transformées à l'étape i) avec ledit bactériophage ;
iii) on place les bactéries infectées à l'étape ii) dans des conditions telles que les bactériophages puissent se répliquer, et qu'au moins une partie des oligonucléotides produits de manière aléatoire puissent s'insérer dans au moins un gène codant une protéine de ciblage dudit bactériophage;
iv) on récolte les bactériophages qui se sont répliqués dans les bactéries infectées à l'étape ii).

### 1) Constructions d'ADN permettant l'insertion d'un oligonucléotide produit de manière aléatoire.

Selon l'invention des oligonucléotides produits de manière aléatoires sont insérés dans les gènes codant les protéines de ciblage des bactériophages. Cette insertion a pour but d'introduire de la variabilité génétique au niveau de certains segments des protéines de ciblage codé par lesdits gènes. Les protéines ainsi traduites, sont susceptibles de présenter des propriétés de reconnaissance ou d'adhésion différentes des protéines initiales du bactériophage.

Les protéines de ciblage du bactériophage se définissent comme des protéines qui participent à la reconnaissance et à l'adhésion du bactériophage à la bactérie hôte. Ces protéines sont de préférence choisies parmi celles constituant les fibres de queue ou de la plate-forme basale du bactériophage T4. Une protéine particulièrement adaptée au procédé selon l'invention est la protéine GP12 de la plate forme basale dont la séquence nucléotidique correspond à SEQ ID N° 1. D'autres protéines de ciblage préférées sont GP36, GP37 ou GP38 constitutives de la partie distale des fibres de queue. Bien entendu, des protéines homologues de celles citées ci-dessus présentes chez d'autres bactériophages sont également préférées. Par séquence homologue, il faut entendre des protéines ayant au moins 50 % d'identité avec ces dernières, de préférence au moins 70 %, plus préférentiellement au moins 90 %.

Préférentiellement, les oligonucléotides produits de manière aléatoire, remplacent par insertion les segments des gènes de protéines de ciblage correspondant aux domaines de la protéine impliqués dans les fonctions d'adhésion ou de reconnaissance. De préférence, ces segments correspondent à des domaines variables, c'est-à-dire à des séquences des domaines de reconnaissance moins conservées d'une espèce de bactériophage à l'autre.

Inversement, il est préférable de conserver les domaines les plus constants des protéines de ciblage en ce qu'en général ils sont nécessaires à la structure tridimensionnelle de ces protéines.

L'exemple est donné dans la présente demande de la modification de la protéine GP12 du bactériophage T4, particulièrement préféré selon l'invention. La protéine GP12 présente un domaine d'ancrage dans lequel il est souhaitable d'introduire de la variabilité tout en maintenant l'intégrité de certains segments notés D (figure 5), lesquels segments correspondent à des domaines conservés chez les protéines homologues des bactériophages de type-T.

Les oligonucléotides destinés à être insérés dans les domaines des protéines choisis, peuvent être produits de manière aléatoire par différentes techniques bien connues de l'homme du métier.

Les oligonucléotides produits de manière aléatoire s'entendent, au sens de la présente invention, comme des séquences nucléotidiques produites de manière artificielle comportant des insertions, des délétions ou des substitutions de nucléotides, qui sont laissées au hasard.

Un des objectifs de l'invention est d'introduire des inserts d'ADN recouvrant le plus grand nombre possible de combinaisons différentes de nucléotides dans les gènes de ciblage sans empêcher le fonctionnement desdits gènes.

Les séquences ainsi produites peuvent être introduites sous forme d'inserts dans les gènes codant les protéines de ciblage des bactériophages par exemple par recombinaison homologue. Ces inserts constituent alors des séquences d'ADN exogènes, produites de manière artificielle.A titre d'exemple, pour synthétiser les oligonucléotides ou inserts selon l'invention, il est possible d'avoir recours à des oligonucléotides de synthèse dont la succession de bases naturelles A, T, C et G a été laissée au hasard, ou bien encore à des oligonucléotides résultant d'une amplification par PCR-erreur (error-prone PCR).

Selon un aspect particulier du procédé, plusieurs oligonucléotides correspondant à différents segments d'un gène ou de plusieurs gènes sont produits de manière aléatoire de sorte à être pourvus d'extrémités 3' ou 5' identiques. De cette manière les oligonucléotides peuvent être permutés ou substitués les uns aux autres dans la séquence modifiée, ce qui permet d'accroître la diversité des séquences génétiques en résultant.

Les oligonucléotides produits aléatoirement peuvent aussi inclure des bases non naturelles, notamment des bases « ambiguës » permettant la traduction d'une seule séquence nucléotidique en des polypeptides différents.

Selon un aspect préféré de l'invention, les oligonucléotides-produits de manière aléatoire sont insérés dans les gènes du bactériophage codant les protéines de ciblage au moyen de la recombinaison homologue.

Pour cela, il est nécessaire d'avoir recours à des constructions d'ADN permettant de réaliser la recombinaison homologue.

De telles constructions dérivent en général de vecteurs décrits dans la littérature, bien connus de l'homme du métier [Sambrook J., Russel D.W. (2001) Molecular Cloning, a Laboratory Manual, CHSL Press].

Néanmoins, pour obtenir des constructions d'ADN utiles aux fins de la présente invention, il est généralement nécessaire d'adapter les vecteurs de recombinaison connus en y clonant une copie du gène ou de la partie du gène modifié que l'on souhaite introduire dans le génome.

Les gènes sont modifiés par introduction de plusieurs oligonucléotides produits de manière aléatoire, de préférence, en des endroits correspondant à des segments variables dudit gène.

A cet égard, notamment dans le cas de la protéine GP12 citée plus haut, il est nécessaire selon l'invention de reconstituer tout ou partie du gène codant la protéine de ciblage désirée en y incluant, aux endroits souhaités, les différentes oligonucléotides produits de manière aléatoire.

Cette reconstitution peut être effectuée de manière classique par reconstitution d'une copie modifiée du gène ou d'une partie de ce gène par clonage, c'est-à-dire en assemblant les oligonucléotides produits de manière aléatoire à des segments conservés de la séquence dans un vecteur. Néanmoins, cette étape peut s'avérer très fastidieuse, en raison des sous clonages et nombreuses étapes de vérifications que cela peut impliquer.

Le présent document propose, dans un de ses aspects particuliers, un procédé visant à faciliter la production d'un insert consistant en la copie d'un gène ou d'une partie d'un gène incluant des oligonucléotides produits de manière aléatoire.

Ce procédé peut s'appliquer à toute séquence nucléotidique dont on cherche à faire varier sélectivement de manière aléatoire certains segments. En effet, ce procédé peut trouver des applications dans de nombreux domaines thérapeutiques. Sa mise en oeuvre est, en effet particulièrement utile dès lors qu'on cherche à modifier le spectre d'activité d'une protéine en modifiant des domaines ciblés de sa séquence génétique.

Ce procédé s'applique plus particulièrement au domaine d'ancrage de gp12 dans lequel on cherche à conserver certains domaines invariants D1 à D4 représentés dans la figure 5.

Ce procédé consiste notamment en un procédé de PCR permettant de muter de manière aléatoire une séquence nucléotidique S, délimitée à ses extrémités 5' et 3' par deux segments F1 et F2, en conservant l'identité d'au moins un segment interne D de ladite séquence nucléotidique. Il comprend les étapes suivantes :
i) on effectue une PCR-erreur sur la totalité de la séquence S à l'aide d'amorces correspondant aux segments F1 et F2, par laquelle la séquence S va être mutée de manière aléatoire sur toute sa longueur;
ii) on élue les produits d'amplification obtenus;
iii) on effectue une PCR haute fidélité à partir des produits d'amplification élués à l'étape ii), en utilisant les couples d'amorces correspondant respectivement à au moins F1 et D, et F2 et D, afin d'amplifier au moins les régions F1-D et D-F2 de S mutées à l'étape i) dont le segment interne D a conservé son identité;
iv) on effectue une PCR haute fidélité à partir des produits d'amplification obtenus à l'étape iii) en utilisant les amorces correspondant à F1 et F2 ;
v) on purifie les produits de PCR obtenus dont la taille correspond à celle de la séquence nucléotidique S.

Une amorce est un acide nucléique simple brin, apte à s'hybrider, en partie ou en totalité, avec l'un des brins d'ADN formant le support ADN double brin de la séquence S. Une amorce est dite « sens » lorsque son enchainement de nucléotides reproduit, à quelques nucléotides prés, une partie du brin d'ADN codant de S. Une amorce est dite « anti-sens » de S, lorsque son enchainement de nucléotides reproduit, à quelques nucléotides prés, une partie du brin complémentaire non codant de S.

Lorsqu'une amorce est donnée comme « correspondant à » un segment donné de la séquence S, cela signifie qu'elle peut être sens ou antisens par rapport à une portion de la séquence S prise sous sa forme de molécule d'ADN double brin.

Le procédé de PCR fait intervenir plus particulièrement des amorces de PCR sens et anti-sens de la manière décrite ci-après et dans les exemples de la présente demande.

Ce procédé est particulièrement avantageux lorsqu'on cherche à conserver l'identité de plusieurs segments internes D_{N} de la séquence nucléotidique S (comme c'est le cas de la protéine GP12). Le procédé comprend alors les étapes suivantes :
i) on effectue une PCR-erreur sur la totalité de la séquence S à l'aide d'au moins deux amorces dont l'une est sens et l'autre antisens respectivement des segments F1 et F2, de sorte à amplifier la séquence S en y introduisant des mutations de manière aléatoire ;
ii) on purifie les produits d'amplification obtenus, lesquels correspondent à des copies de S mutées de manière aléatoire;
iii) on effectue une PCR haute fidélité à partir des produits d'amplification purifiés à l'étape ii), en utilisant comme couples d'amorces sens et antisens respectivement au moins :
   - une amorce sens correspondant à F1 et une amorce anti-sens de D_{N}, afin d'amplifier au moins la région F1- D_{N} des copies de S mutées, et
   - une amorce sens correspondant à D_{N} et une amorce antisens de F2, afin d'amplifier au moins la séquence D_{N}-F2 des copies de S mutées;
iv) on purifie les produits d'amplification obtenus à l'étape iii), lesquels consistent en des copies des segments F1-D_{N} et D_{N}-F2 de la séquence S mutée à l'étape i), dans lesquels la séquence du segment D a conservé son identité ;
v) on effectue une PCR haute fidélité à partir des produits d'amplification obtenus à l'étape iv) en utilisant au moins un couple d'amorces sens et antisens de F1 et F2 ;
vi) on purifie les produits de PCR obtenus à l'étape v), lesquels correspondent à des séquences nucléotidiques dudit insert mutées de manière aléatoire dans lesquels au moins la séquence du segment D_{N} a conservé son identité.

Par PCP-erreur, on entend une réaction de polymérisation en chaîne effectuée dans des conditions ne permettant pas une réplication fidèle des séquences d'ADN. Ce type de réaction peut être obtenu en mettant en oeuvre une polymérase classique de type *Taq* en conditions faiblement stringentes et en présence de sels de manganèse, ainsi que cela est décrit dans la littérature [Cadwell, R.C.et al., Randomization of genes by PCR mutagenesis, PCR Methods Appl.,1992, 28-33].

Une PCR fidèle est, au contraire, une réaction de polymérisation en chaîne permettant d'amplifier une matrice d'ADN avec un taux d'erreur de réplication très faible. Ce type de réaction peut être obtenu en mettant en oeuvre, par exemple, une polymérase de type *Pfu* en condition stringente, ainsi que cela est décrit dans la littérature [Inmis, M.A. et Eds., PCR Protocols : a guide to methods and applications, 1989, Academic Press].

Avantageusement, les segments des gènes que l'on cherche à diversifier servent eux-mêmes de matrice pour produire les oligonucléotides par PCR erreur. Les produits d'amplifications obtenus selon ce procédé particulier constituent une banque de séquences nucléotidiques S diversifiées de manière aléatoire dont les domaines internes D_{N} ont été conservés, lesquelles séquences comprennent :
- l'insertion d'un ou plusieurs oligonucléotides produits de manière aléatoire ; et
- des segments de séquence dudit gène ayant conservé leur identité.

En général les segments internes D_{N} conservent, plus de 50 % d'identité par rapport à leur séquence initiale dans la protéine sauvage, de préférence plus de 70 %, et plus préférentiellement plus de 90 %, voire plus de 99 % selon les conditions de PCR utilisées.

Une fois traduites en protéine, les séquences nucléotidiques contenues dans la banque susvisée peuvent résulter en l'expression de protéines présentant une diversité de séquences polypeptidiques produites de manière aléatoire incluant des domaines internes D_{N} dont la séquence est conservée.

Ces séquences peuvent être directement clonées dans des vecteurs d'expression, ou plus préférentiellement dans des vecteurs de recombinaison homologue, dont l'homme du métier dispose.

Il est possible grâce au procédé décrit ci-dessus de modifier le génome d'un bactériophage à l'aide de vecteurs de recombinaison homologue diversifiés incluant des variants aléatoires d'un même gène, de préférence un gène codant une protéine de ciblage d'un bactériophage.

Les constructions d'ADN comprennent de préférence :
- une région permettant la duplication de ladite construction dans une bactérie hôte ;
- une région permettant la recombinaison homologue dans le génome du bactériophage au niveau d'un gène codant une protéine de ciblage, ladite région comprenant deux séquences d'ADN homologues aux séquences dudit gène codant une protéine de ciblage, lesquelles délimitent un segment d'insertion incluant un oligonucléotide dont la séquence est produite de manière aléatoire, de préférence selon le procédé par PCR décrit ci-dessus.

Selon un aspect préféré de l'invention, la région permettant la recombinaison homologue comprend tout ou partie du gène codant la protéine de ciblage, de préférence la totalité de la séquence du gène.

De préférence, cette seconde région consiste en un produit d'amplification susceptible d'être obtenu selon le procédé de mutation aléatoire exposé ci-dessus.

Les constructions d'ADN, prises dans leur ensemble, permettent d'intégrer dans le génome du bactériophage un très grand nombre de séquences nucléotidiques différentes codant des protéines de ciblage modifiées.

Selon un aspect préféré de l'invention plusieurs gènes codant des protéines de ciblage du bactériophage sont mutés simultanément par recombinaison homologue selon le procédé de l'invention. Pour parvenir à un tel résultat, l'invention prévoit de transformer la bactérie hôte successivement à l'aide de vecteurs différents, ciblant chacun un gène différent.

Les vecteurs préférés permettant de modifier simultanément les gènes GP12, GP37 ou GP38 du bactériophage T4 selon l'invention dans la bactérie hôte E.coli sont, par exemple, les vecteurs pACYC184 (ATCC 37033), pBAD18-K (ATCC 87397) et RR1 (ATCC 87076). De tels vecteurs présentent l'avantage de posséder des marqueurs conférant une résistance à des antibiotiques différents et ne partagent pas de séquences nucléotidiques communes susceptibles d'occasionner des recombinaisons entre les différents vecteurs une fois ceux-ci intégrés dans la bactérie hôte.

### 2) Transformation de la bactérie hôte

Un bactériophage selon l'invention est, de préférence, un bactériophage lytique, naturel ou modifié.

De préférence, le bactériophage utilisé est un bactériophage de type-T, tels que les bactériophages T4, T5, T6 et T7, bien connus de l'homme du métier et plus particulièrement le bactériophage T4, dont le génome a été séquencé [Miller, E.S. et al., Bactériophage T4 genome, Microbiol Mol. Biol. Rev., 2003, 67(1):86-156]. La séquence complète du génome du bactériophage est disponible dans Genbank (AF 158101).

Une bactérie hôte est une bactérie utilisée couramment pour répliquer le bactériophage que l'on cherche à modifier. De préférence, la bactérie hôte est une souche que l'on peut transformer à l'aide d'une construction d'ADN permettant de modifier le bactériophage par recombinaison homologue.

Une bactérie hôte particulièrement adaptée aux bactériophages de type-T pour la mise en oeuvre du procédé de la présente invention, est *Escherichia coli,* par exemple la souche DK8 (ATCC 47038).

Dans le cadre de la modification des bactériophages de type-T, il est avantageux d'utiliser une souche d'*E.coli* transformée à l'aide d'un vecteur de type Mini-lambda, dérivé du prophage lambda et comprenant les gènes *exo, bet* et *gam.* Un tel vecteur permet de contrôler, par exemple, la recombinaison homologue en fonction de la température à laquelle la bactérie hôte est cultivée et ainsi limiter les événements de recombinaison aux seules étapes de réplication prévues selon l'invention.

Les techniques de recombinaison homologue utilisées pour la mise en oeuvre de la présente invention sont connues de l'homme du métier. Elles sont décrites par exemple dans [Poteete, A.R. et al., FEMS Microbiol. Lett, 2001, 201(1) :9-14 ; Kuzminov, A. et al. PNAS, 2001, 98(15) :8298-305].

La bactérie hôte est donc transformée de préférence à l'aide d'une construction d'ADN permettant de modifier un gène codant une protéine de ciblage de bactériophage, comprenant un insert correspondant à une copie mutée de manière aléatoire de tout ou partie du gène codant une protéine de ciblage.

Cet insert est, de préférence, produit par le procédé de PCR décrit précédemment.

L'étape de transformation de la bactérie hôte selon le procédé de l'invention peut intervenir en une ou plusieurs fois. Lorsque plusieurs vecteurs sont utilisés pour modifier des gènes différents de protéines de ciblage, il est avantageux de procéder séquentiellement, c'est-à-dire en introduisant les vecteurs les uns après les autres. In est alors possible de conserver les bactéries transformées à chaque étape. Il est ainsi toujours possible de revenir à l'étape précédente pour réintroduire un nouveau vecteur à la place de celui introduit en dernier. De nombreuses combinaisons de vecteurs peuvent ainsi être testés sans avoir à réintroduire tous les vecteurs dans la bactérie hôte.

Au terme de l'étape de transformation, on dispose d'une ou plusieurs banques de bactéries hôte transformées à l'aide d'une ou plusieurs des constructions.

Chacune des bactéries de cette banque contient potentiellement une construction capable de transformer par recombinaison homologue une ou plusieurs des protéines de ciblage du bactériophage de manière différente.

Un objet du présent document consiste donc en une banque de bactéries hôtes transformée à l'aide des constructions d'ADN décrites ci-dessus, susceptible d'être infectée à l'aide d'un bactériophage.

Une telle banque de bactéries hôte présente l'avantage de pouvoir être multipliée et conservée. Elle constitue un produit intermédiaire renouvelable utile pour la production des bactériophages recombinants décrits ci-après.

### 3) Infection et réplication du bactériophage dans les bactéries hôte transformées

L'étape d'infection des bactéries hôtes par le bactériophage sélectionné ne pose aucune difficulté particulière dans la mesure où le bactériophage n'est pas modifié à ce stade. L'infection est initiée de préférence durant la phase exponentielle de croissances des bactéries hôtes, à environ 30°C.

C'est ensuite au cours de l'étape de réplication du génome du bactériophage dans la bactérie que la recombinaison homologue peut s'effectuer entre la région homologue du gène incluant l'oligonucléotide produit de manière aléatoire et le gène présent dans le génome du bactériophage.

Cette étape de réplication débute immédiatement après l'infection des bactéries hôte par les bactériophages.

Selon un aspect préféré de l'invention les bactériophages sont introduits dans la culture des bactéries hôtes transformées en même temps qu'un choc thermique entre environ 40 et 45°C est appliqué. Cette température est maintenue pendant une durée limitée comprise entre 5 et 15 minutes, de préférence entre 12 et 15 minutes, ce qui permet d'activer la recombinaison homologue. Les bactéries hôtes étant préférentiellement transformées à l'aide d'un vecteur Mini-lambda permettant de conditionner la recombinaison homologue à certaines conditions de température, notamment en cas de choc thermique, la recombinaison homologue se produit sur une durée équivalente ou inférieure à celle d'un cycle de réplication. Les bactéries hôtes sont ensuite transférées dans une culture à une température normale d'environ 30°C à laquelle la recombinaison homologue se produit beaucoup plus difficilement. Cette seconde période à environ 30°C dure approximativement 25 minutes, soit une durée équivalant à un seul cycle de réplication.

Un aspect particulièrement innovant du procédé selon l'invention réside dans le fait qu'on récolte les bactériophages à l'issue de la seconde période de culture, au terme d'un seul cycle de réplication, en moyenne, de préférence avant qu'un second cycle de réplication des bactériophages ne se produise.

Cette manière de procéder présente l'avantage de récolter les bactériophages recombinants, avant qu'un nouveau cycle de réplication dans la bactérie hôte ne se produise.

En se limitant ainsi à un seul cycle de réplication, les bactériophages n'ont pas le temps de réinfecter les bactéries hôte pour un second cycle de réplication. On peut ainsi récolter les bactériophages modifiés ayant perdu la capacité de reconnaître ou infecter leur bactérie hôtes, lesquels autrement seraient éliminés au cours des cycles suivants de réinfection.

### 4) Banques de Bactériophages recombinants

Le procédé visé par la présente invention permet d'obtenir un ensemble de bactériophages recombinants très diversifié.

Ces bactériophages forment une banque de bactériophages.

Une banque de bactériophages recombinants se compose donc de variants polynucléotidiques comportant des copies différentes d'au moins un gène codant une protéine de ciblage, ledit gène comprenant l'insertion d'une séquence oligonucléotidique produite de manière aléatoire.

De préférence, ledit gène dont les bactériophages comportent des copies différentes code pour la protéine de ciblage GP12.

Les bactériophages qui composent cette banque peuvent également comporter des copies différentes de plusieurs gènes codant des protéines de ciblage, lesdits gènes comprenant l'insertion de séquences oligonucléotidiques produites de manière aléatoire. Ces différents gènes peuvent être modifiés dans les mêmes bactériophages ou bien dans des bactériophages différents de la même banque. De préférence, plusieurs gènes de ciblage sont modifiés dans chaque bactériophage composant la banque de bactériophages. Les protéines de ciblages modifiées sont de préférence, GP12, GP36, GP37, GP38 ou des protéines homologues de celles-ci. Afin qu'une banque de bactériophages puisse recouvrir le plus grand nombre possible de bactériophages différents, il convient de mettre en oeuvre un nombre suffisant de bactéries hôtes transformées car c'est ce nombre qui détermine le nombre de bactériophages récoltés.

Si ce nombre est suffisant, une banque de bactériophages selon l'invention comprend au minimum, au moins 10³, de préférence 10⁵, plus préférentiellement 10⁸ variants différents d'un même bactériophage, lesdits variants se distinguant par la séquence d'au moins une de leurs protéines de ciblage.

Le procédé selon l'invention vise en effet, en premier lieu, à augmenter la diversité des protéines de ciblages exprimées par les bactériophages et empêche l'élimination des bactériophages recombinants, notamment ceux qui ne se seraient pas ou moins vite répliqués dans la bactérie hôte au cours des cycles successifs de réplication.

La diversité des bactériophages dans la banque peut se justifier par un calcul simple de dénombrement.

Ainsi, si on considère que préférentiellement :
- 3 gènes codant des protéines de ciblages sont modifiés ; par l'insertion d'au moins 3 oligonucléotides composés de séquences aléatoires d'au moins 12 nucléotides ; et que
- 1/3 des séquences nucléotidiques imposent une modification polypeptidique au niveau des protéines de ciblage ; et que
- seulement 3 des 4 bases (A, T, C, G) sont susceptibles de produire une mutation par rapport à la protéine initiale ;

On obtient alors un minimum de 3²⁴ possibilités de mutations au niveau polypeptidique, ce qui revient à dénombrer quelques 2,8.10¹¹bactériophages potentiellement différents.

Le présent document vise plus particulièrement un bactériophage recombinant isolé, issu de la banque de bactériophage, capable d'infecter une bactérie cible et de s'y multiplier.

Un tel bactériophage peut être obtenu en procédant selon les étapes suivantes :
i) on introduit dans au moins un gène codant une protéine de ciblage d'un bactériophage, un oligonucléotide dont la séquence est produite de manière aléatoire, ainsi que cela est décrit plus haut ;
ii) on met en contact les bactériophages génétiquement modifiés obtenus selon l'étape i) avec une cellule dont l'identité est connue ou inconnue;
iii) on sélectionne au moins un bactériophage mis en contact à l'étape ii) capable d'infecter ladite cellule dont l'identité est connue ou inconnue.

Afin de faciliter la sélection des bactériophages recombinants capables d'infecter les bactéries dont l'identité est connue ou inconnue, il est avantageux de disposer de bactériophages détectables par fluorescence ou luminescence.

A cet égard le bactériophage utilisé pour infecter les bactéries hôtes peut être un bactériophage lui-même préalablement modifié, rendu fluorescent par l'introduction d'un gène codant une protéine luminescente ou fluorescente.

Le bactériophage comprend une protéine de capside fusionnée à une protéine luminescente ou fluorescente comme par exemple la GFP (Green Fluorescent Protein).

De préférence la protéine Hoc [Genbank NP 049793] de la capside du bactériophage, ou bien une partie de celle-ci, est fusionnée à cette protéine luminescente ou fluorescente. Cette fusion reste normalement sans effet sur les propriétés de reconnaissance et d'adhésion du bactériophage à la bactérie. La présence d'une protéine fluorescente permet de localiser les bactéries qui, du fait de leur infection, recèlent de nombreux bactériophages. La concentration des bactériophages à leur endroit permet d'atteindre un niveau de fluorescence pouvant être détecté par les instruments de mesure connus de l'homme du métier. Ces bactériophages modifiés s'avèrent particulièrement utile pour cribler les bactériophages infectieux lorsque les bactéries sont cultivées de manière étalées sur un support solide.

Ce document vise également une protéine de ciblage modifiée extraite d'un bactériophage recombinant selon l'invention ou traduite à partir de l'ADN provenant dudit bactériophage.

Le présent document couvre également tout procédé pour obtenir des protéines de ciblage modifiées de bactériophage caractérisé en ce qu'on met en oeuvre un bactériophage recombinant selon l'invention.

### 5) Utilisation des bactériophages recombinants obtenus

Une banque de bactériophages peut être utilisée pour lutter contre des bactéries connues ou inconnues. En effet, la diversité des bactériophages recombinants présents dans la banque ayant des protéines de ciblages différentes est telle que la probabilité que l'un de ces bactériophages puisse infecter la bactérie connue ou inconnue est significativement non nulle.

Une bactérie inconnue est une bactérie n'ayant pas encore été caractérisée, ou du mois pas suffisamment pour pouvoir établir, par exemple, son antibiogramme. Il peut s'agir d'une bactérie émergente contre laquelle il n'existe aucun antibiotique actif ou bien encore une bactérie dont l'existence n'est pas encore établie.

Une banque de bactériophages peut avantageusement être utilisée de manière préventive, à l'encontre de bactéries connues ou inconnues susceptibles de se développer à la surface d'un objet ou chez les êtres vivants.

Ce document porte donc également sur un procédé d'aseptisation caractérisé en ce qu'on met en oeuvre une banque de bactériophages comprenant des bactériophages modifiés par insertion, dans un ou plusieurs gènes codant des protéines de ciblage de ce bactériophage, un oligonucléotide produit de manière aléatoire.

Un autre objet concerne une composition solide ou liquide comprenant une banque de bactériophage, par exemple, une poudre pouvant être pulvérisée sur une surface que l'on cherche à rendre exempte de bactéries.

Une application préférée consiste à traiter les dispositifs de climatisation, notamment les systèmes aéroréfrigérants à l'aide des banques de bactériophages.

Une telle composition peut être utilisée également en tant que médicament, notamment pour prévenir ou traiter des infections bactériennes et plus particulièrement pour traiter les affections des voies respiratoires ou cutanées.

Un bactériophage recombinant, qui est identifié comme capable d'infecter une bactérie donnée, peut être utilisé seul dans une des applications décrites ci-dessus.

Un bactériophage recombinant selon l'invention peut être utilisé en tant que bactériophage de référence pour la détection et/ou l'identification de la bactérie connue ou inconnue qu'il est capable d'infecter.

Un bactériophage de référence, est un bactériophage rendu capable de cibler un type de bactérie donné, de sorte à pouvoir servir en tant qu'outil de détection ou de diagnostic.

Les bactériophages recombinants sont en général obtenus à l'aide de souches bactériennes de laboratoires faciles à cultiver. Ils peuvent donc s'avérer utiles vis-à-vis de bactéries qui, au contraire, sont difficiles à cultiver dans les conditions de laboratoire.

Un bactériophage recombinant peut donc être mis en oeuvre dans des méthodes de diagnostic, par exemple des méthodes comprenant la mise en contact dudit bactériophage avec des échantillons provenant de patients chez lesquels une infection bactérienne est à rechercher.

Selon un aspect préféré, les bactériophages sont fluorescents ou luminescent, ce qui permet un suivi en temps réel de l'évolution d'une infection, notamment en ce qu'on peut tracer les bactéries à l'aide de leurs bactériophages respectifs.

Les utilisations et méthodes décrites ci-dessus sont particulièrement utiles dans des situations d'urgence pour détecter ou lutter contre une bactérie émergente ou résistante.

Les exemples qui suivent ont pour but d'illustrer l'invention sans en limiter la portée.

### Préparation des séquences de variants gp12, gp37 et gp38 de bactériophages T4

Ce qui suit décrit le mode opératoire utilisé pour gp12, mais il peut être transposé sans difficulté pour l'homme du métier à la modification d'autres gènes codant des protéines de ciblage, tels que gp37 et gp38.

### Etape 1 : Préparation du gène gp12

Le gène gp12 est amplifié par PCR à partir de l'ADN génomique du T4 de type sauvage obtenu à partir d'une culture concentrée de lysat de bactériophages (voir ci-dessus) en utilisant comme amorces
gp12F 5'-TGAGTAATAATACATATCAACACG (SEQ ID No.2), est
gp12R 5'-TGATTCTTTTACCTTAATTATGTAC (SEQ ID No.3).

Après purification sur un gel d'agarose préparatif, le produit de PCR (gp12A) est utilisé comme matrice dans des réactions PCR sensibles à l'erreur avec pour but d'introduire des mutations et insertions ponctuelles dans la région codante correspondant au domaine de liaison au récepteur de gp12 (voir figure 5).

### Etape 2 : Introduction de mutations aléatoires dans le domaine de liaison au récepteur.

Une série de 4 réactions PCR emboîtées (40 cycles chacune) est mise en oeuvre en présence de Mn2 de manière à induire des erreurs de polymérase aléatoires.

Les PCR-erreur sont conduites dans un volume réactionnel de 100 µl, en utilisant des matrices et amorces à des concentrations finales respectivement égales à 400 ng et 30 pmol, avec 0,2 mM de dATP et dGTP (de chacun), 1 mM de dCTP et dTTP (de chacun), 2,5 mM de MgCl₂, 0,7 mM de MnCl₂ et 5 U d'ADN-polymérase *Taq* (New England Biolabs, Inc.) dans du tampon réactionnel 1×. La PCR est effectuée à 96 °C pendant 2 min, avec 30 cycles à 95 °C pendant 1 min, 56 °C pendant 1 min et 72 °C pendant 2 min, et une extension finale à 72 °C pendant 7 min.

La première réaction (P1-1) utilise comme amorces :
p12NF1F 5'-TCAAGGTAACCGCATCGTAAC (SEQ ID No.4)
p12NF2R 5'-AAAGACCACGCATGTCAG (SEQ ID No.5)

La deuxième réaction (P2-1) utilise comme amorces :
p12NF2F 5'-TGCCATGGTGGAACTGTTCA (SEQ ID No.6)
p12NF3R 5'-CACCTAATCTAGGTTTAC (SEQ ID No.7)

La troisième réaction (P3-1) utilise comme amorces :
p12NF3F 5'-CTGACATGCGTGGTCTTT (SEQ ID No.8)
p12NF4R 5'-ATGTTTATGATAAGACAT (SEQ ID No.9)

La quatrième réaction (P4-1) utilise comme amorces :
p12NF4F 5'-GTAAACCTAGATTAGGTG (SEQ ID No.10)
p12NF5R 5'-TCATTCTTTTACCTTAATTAT(SEQ ID No.11)

Chacun de ces produits de réaction présente un chevauchement partiel avec deux autres produits de réaction et les amorces utilisées correspondent à des domaines invariants conservés dans la protéine gp12. Ces domaines doivent être préservés fidèlement dans les structures de gènes mutés finales produites. Cependant, certains des fragments produits par les réactions PCR sensibles à l'erreur peuvent parfaitement avoir subi une mutagenèse dans ces régions. Afin de préserver des domaines intacts, chacun des produits précités doit alors être soumis à des réactions PCR à haute fidélité ayant pour but d'amplifier sélectivement seulement les fragments dans lesquels le domaine conservé a été retenu.

### Etape 3 : Amplification sélective des fragments désirés.

Celle-ci est effectuée par deux séries de réactions PCR à haute fidélité comprenant chacune 25 cycles.

Les PCR haute fidélité sont conduites dans un volume réactionnel de 50 µl, en utilisant des matrices et amorces à des concentrations finales respectivement égales à 250 ng et 40 pM, dans du tampon pour *pfu* 1× (Tris-HCl 20 mM à pH 9,0, KCI 10 mM, MgSO₄ 1 mM, (NH₄)SO₄ 6 mM, 0,1 % de Triton X-100, 0,1 mg/ml de SAB) avec 200 µM de dNTP et 5 U de polymérase *pfu* (Promega). Les profils de PCR sont les suivants : 94 °C pendant 20 s, 45 °C pendant 15 s et 72 °C pendant 30 s, avec répétition pendant 20 cycles.

Dans la première série de réactions (P1-2 à P4-2), une portion aliquote (environ 250 ng) de chacun des produits de PCR précités est amplifiée en utilisant les amorces « F » correspondantes (par exemple : p12NF1F) biotinylées à l'extrémité 5'. Cela est nécessaire pour séparer les produits désirés des matrices non amplifiables (domaines conservés mutés) qui ont des longueurs pratiquement identiques et qui ne peuvent être séparées par électrophorèse sur gel.

Les produits de chaque réaction sont ensuite passés sur des mini-colonnes d'exclusion dimensionnelle (pour éliminer l'excès d'amorces), purifiés individuellement sur des billes de streptavidine, lavés dans du tampon de liaison, élués, précipités et remis en suspension dans ddH₂O.

Dans la seconde série de réactions, une portion aliquote des produits de réaction P1-2 purifiés est mélangée à une quantité égale de produits de réaction P2-2. Ceux-ci ont en commun le site d'amorçage F2-R1 et F3-R2. En conséquence, le brin « - » des produits de réaction P2-2 sert d'amorce pour le brin « + » des produits de réaction P1-2 et l'extension avec une polymérase débute au site d'amorçage F2-R1 interne de P1-2. L'extension échoue pour les produits de réaction P1-2 où ce site conservé a été muté de manière significative.

De manière similaire, le brin « + » des produits de réaction P1-2 sert d'amorce pour le brin « - » des produits de réaction P2-2 et l'extension avec une polymérase débute au site d'amorçage F3-R2 interne de P2-2. L'extension échoue pour les produits de réaction P2-2 où ce site conservé a été muté de manière significative.

Les produits de PCR amplifiés avec succès (PA-1) correspondent à une fusion des fragments P1-2 et P2-2 dans laquelle chacun des quatre domaines conservés (F1 + F2-R1 + F3-R2 + F4-R3) a été retenu à l'état non muté.

Un mélange similaire est préparé à partir des produits de réaction P3-2 et P4-2. Dans ces réactions, une extension couronnée de succès est basée sur des sites conservés internes F4-R3 (P3-2) et F5-R4 (P4-2) intacts et les produits de PCR résultants (PA-1 et PB-1) correspondent à une fusion des fragments P3-2 et P4-2 dans laquelle chacun des quatre domaines conservés (F3-R2 + F4-R3 + F5-R4 + R5) a été obtenu à l'état non muté.

Les produits obtenus sont purifiés par électrophorèse sur gel préparatif puisque les dimensions des produits de PCR fusionnés sont très différentes de celles des matrices individuelles.

Afin d'augmenter le rendement dans cette seconde série de réactions PCR, le protocole d'extension peut être modifié de la manière suivante :
10 cycles avec seulement les produits P1-2 et P2-2, et P3-2 et P4-2 dans les mélanges réactionnels.

Les réactions sont interrompues, 50 ng de chacune des amorces p12NF1F et p12NF3R sont ajoutés au mélange PA-1 tandis que 50 ng de chacune des amorces p12NF3F et p12NF5R sont ajoutés au mélange PB-1, en laissant ensuite les réactions PCR se poursuivre pendant 15 cycles supplémentaires.

### Etape 4 : Reconstitution du domaine d'ancrage muté de gp12.

Après purification, des portions aliquotes égales des produits PA-1 et PB-1 sont utilisées ensemble dans une réaction PCR à haute fidélité comprenant 30 cycles ayant pour but de reconstituer le domaine total de liaison au récepteur de gp12 en fragments uniques renfermant les diverses mutations introduites ci-dessus.

Dans ce cas, comme dans la série précédente de réactions, les produits PA-1 servent d'amorces d'extension pour les produits PB-1 et *vice versa.*

Le produit de PCR fusionné final (gp12BD-Fu) est purifié par électrophorèse sur gel puisque ses dimensions sont très différentes de celles des matrices individuelles.

Afin d'augmenter le rendement, le protocole d'extension peut être modifié en procédant à une réaction comprenant 15 cycles avec seulement les produits P1-2 et P2-2, et P3-2 et P4-2 dans les mélanges réactionnels. Les réactions sont interrompues, 50 ng de chacune des amorces p12NF1F et p12NF5R sont ajoutés au mélange, puis on laisse ensuite les réactions PCR se poursuivre pendant 15 cycles supplémentaires.

Après purification, une portion aliquote (environ 250 ng) du domaine de liaison au récepteur de gp12 reconstitué est utilisée comme matrice dans une nouvelle série de quatre réactions PCR sensibles à l'erreur, avec ensuite des amplifications sélectives et des reconstitutions des domaines de la manière décrite ci-dessus.

Cependant, puisque, à chaque étape de fusion (étapes 3 et 4 ci-dessus), toutes les mutations possibles introduites individuellement dans chaque sous-domaine sont brassées de manière aléatoire dans le domaine reconstitué final de liaison au récepteur, il existe des limites quant au nombre de cycles de mutagenèse qui peuvent être effectués sans effet négatif sur les domaines conservés. En raison des étapes nécessaires d'amplification sélective, il est estimé que, au-delà de 4 cycles successifs, toutes les mutations nouvellement introduites présenteront une forte probabilité d'effet négatif sur un domaine conservé ou d'introduction d'une réversion restituant la séquence T4 originale, non mutée.

### Etape 5 : Reconstitution de la copie modifiée du gène gp12 dans son entier

### 1) Amplification du segment de gp12 en amont du domaine de liaison au récepteur.

Le gène gp12, engendré par PCR, produit ci-dessus (gp12A) est utilisé comme matrice conjointement avec les amorces
gp12F 5'-TGAGTAATAATACATATCAACACG (SEQ ID No.12), et
gp12AR 5'-GTTACGATGCGGTTACCTTGT (SEQ ID No.13)
Il s'ensuit une purification des produits d'amplifications obtenus sur un gel d'agarose préparatif, une précipitation puis une remise en suspension dans ddH₂O.

### 2) Amplification du domaine de liaison muté de gp12

Une portion aliquote (environ 500 ng) du produit de PCR (gp12B) est utilisée conjointement avec une quantité égale de domaine de liaison au récepteur de gp12 reconstitué (gp12BD-Fu) dans une réaction PCR à haute fidélité. Il faut noter que ces deux produits présentent seulement en commun un chevauchement de 20 pb. En conséquence, le profil de la réaction PCR doit être modifié pour prendre ce fait en considération.

Profil de PCR modifié : A) 30 min à 96 °C puis 5 cycles d'1 min à 94 °C, de 10 s à 45 °C, et ensuite B) 5 cycles d'1 min à 94 °C et de 20 s à 50 °C, puis C) 5 cycles d' 1 min à 94 °C, de 30 s à 50 °C, et finalement D) 15 cycles d' 1 min à 94 °C, d'1 min à 55 °C, de 5 secondes à 72 °C.

Afin d'augmenter le rendement, la réaction peut être interrompue à la sous-étape D ci-dessus et les amorces gp12F et p12NF5R peuvent être introduites dans le mélange. On laisse ensuite la réaction se rétablir et s'effectuer jusqu'à son terme.

Les produits finals (gp12-Mut) sont purifiés sur un gel d'agarose préparatif, précipités et remis en suspension dans ddH₂O jusqu'à leur utilisation.

### Etape 6 : Vérification par séquençage des produits de PCR gp12-Mut obtenus

Les produits de PCR purifiés sur gel d'agarose sont séquencés à l'aide d'un séquenceur automatique afin de vérifier que des mutations ont bien été introduites de manière aléatoire dans gp12, tout en conservant intacte les domaines D1 et D2. Le séquençage est effectué sur deux portions correspondant respectivement aux nucléotides 1089 à 1110 (Fig. 6A) et 1195 et 1212 (Fig. 6B) de gp12 (SEQ ID No.1). Les profils de séquençage typiquement obtenus sont présentés dans la Fig.6. Lorsqu'on compare leurs profils à ceux de la séquence initiale de gp12, on peut constater que de nombreux pics supplémentaires viennent se superposer au profil attendu de gp12. Ces pics supplémentaires traduisent une fréquence élevée de mutations introduites dans la séquence gp12 par le procédé de PCR. Ces pics ne se retrouvent pas au niveau du domaine D1, ce qui confirme que ce domaine a conservé son identité de séquence avec gp12.

### Clonage du gène gp12 muté dans un vecteur alternatif pour recombinaison homologue

Les modes opératoires précités ont permis d'engendrer, de façon similaire à gp12, les gènes mutants gp37 et gp38 de T4.

Le but des modes opératoires suivants est alors d'introduire ces gènes de T4 variants de manière à favoriser leur introduction, par recombinaison homologue, dans le génome du bactériophage T4 de manière à produire des descendances de T4 ayant des spécificités de ciblage d'hôtes différentes de celle du bactériophage parental.

A cet effet, les gènes mutés engendrés ci-dessus doivent être introduits dans des vecteurs alternatifs (un vecteur différent pour chaque gène). Dans le cas présent, les vecteurs pACYC184, pBAD18-K et RR1 sont utilisés pour cloner chacun des gènes mutés.

Le vecteur choisi (par exemple pACYC184 pour le clonage de gp12Mut) est coupé au niveau du site Sma I, purifié et remis en suspension dans 20 µl de ddH₂O et mélangé au produit de PCR gp12Mut en un rapport de 1:3. Après ligation, l'ADN est purifié, remis en suspension dans ddH₂O et transformé dans des cellules DK8 (ATCC 47038) « électrocompétentes ». Ce vecteur porte un gène de résistance au chloramphénicol (Chl). Ainsi, après une électroporation et une période de rétablissement de 1 h, les cellules sont transférées à 10 ml de milieu LB contenant 170 µg/ml de chloramphénicol et sont cultivées à 30 °C pendant 4 h avec aération constante. Puis les cellules sont étalées sur des plaques de milieu LB + Chl et cultivées pendant une nuit à 30 °C. Quelques colonies sont prélevées pour la vérification par PCR de la présence du segment d'insertion gp12Mut et quelques colonies positives sont cultivées dans du milieu LB + Chl pour la préparation d'une culture concentrée de cellules (DK8-p12C).

### Introduction des gènes mutés gp37 et gp38 dans la bactérie hôte

Une culture fraîche d'une nuit de DK8-p12C est utilisée pour préparer des cellules électrocompétentes.

Le vecteur pBAD18-K est coupé au niveau du site Sma I, purifié et remis en suspension dans 20 µl de ddH₂O et mélangé au produit de PCR gp37Mut en un rapport de 1:3. Après ligation, l'ADN est purifié, remis en suspension dans ddH₂O et transformé dans des cellules DK8-p12C « électrocompétentes ».

Le vecteur utilisé dans ce cas porte un gène de résistance à la kanamycine (Kan). Ainsi, après une électroporation et une période de rétablissement de 1 h, les cellules sont transférées à 10 ml de milieu LB contenant 170 µg/ml de chloramphénicol et 50 µg/ml de kanamycine et sont cultivées à 30 °C pendant 4 h avec aération constante. Puis les cellules son étalées sur des plaques de milieu LB + Chl + Kan et cultivées pendant une nuit à 30 °C. Quelques colonies sont prélevées pour la vérification par PCR de la présence des segments d'insertion gp12Mut et gp37Mut et quelques colonies positives sont cultivées dans du milieu LB + Chl + Kan pour la préparation d'une culture concentrée de cellules (DK8-p12C-p37K).

Une culture fraîche d'une nuit de DK8-p12C-p37K est utilisée pour préparer des cellules électrocompétentes. Le vecteur RR1 est coupé au niveau du site Sma I, purifié et remis en suspension dans 20 µl de ddH₂O et mélangé au produit de PCR gp37Mut en un rapport de 1:3. Après ligation, l'ADN est purifié, remis en suspension dans ddH₂O et transformé dans des cellules DK8-p12C-p37K « électrocompétentes ». Le vecteur utilisé dans ce cas porte un gène de résistance à l'ampicilline (Amp). Ainsi, après une électroporation et une période de rétablissement de 1 h, les cellules sont transférées dans 10 ml de milieu LB contenant 170 µg/ml de chloramphénicol + 50 µg/ml de kanamycine + 60 µg/ml d'ampicilline et sont cultivées à 30 °C pendant 4 h avec aération constante. Puis les cellules sont étalées sur des plaques de milieu LB + Chl + Kan + Amp et cultivées pendant une nuit à 30 °C. Quelques colonies sont prélevées pour la vérification par PCR de la présence des segments d'insertion gp12Mut, gp37Mut et gp38Mut et quelques colonies positives sont cultivées dans du milieu LB + Chl + Kan + Amp pour la préparation d'une culture concentrée de cellules (DK8-p12C-p37K-P38A).

Il reste à présent à construire un hôte capable de présenter une puissance de recombinaison extrêmement efficace.

### Construction de bactérie hôtes E. coli « mini-λ »

Pour obtenir la recombinaison efficace de l'ADN donneur dans des fonds *recA*⁺ ou *recA⁻,* on prépare l'hôte *E*. *coli* qui contient un prophage λ portant les gènes de recombinaison *exo*, *bet* et *gam* sous le contrôle d'un répresseur cl de λ sensible à la température. Les gènes exo, *bet* et *gam* peuvent être aisément actionnés à 42 °C et inhibés à 32 °C. Lorsque les fonctions de λ sont actionnées pendant un temps réduit à 5 min, les cellules deviennent plus recombinogènes et absorbent l'ADN linéaire sans sa destruction. λ Gam inhibe l'attaque de l'ADN linéaire par la nucléase RecBCD de *E*. *coli,* et Exo et Beta engendrent une activité de recombinaison pour cet ADN linéaire. De manière plus importante, cette recombinaison est efficace avec des homologies d'ADN limitées à 30 à 50 pb aux extrémités des substrats consistant en ADN linéaire.

Les oligonucléotides 5' GTATGCATGCTGGGTGTGG (MλRf) et 5' CGCACTCTCGATTCGTAGAGCCTCG (MλRr) sont utilisés comme amorces pour l'amplification par PCR de l'ADN provenant de la région *attP-cro* en utilisant l'ADN de λ *cl857* comme matrice.

Une fois le prophage λ engendré par PCR, une possibilité consiste à le cloner le prophage λ dans le plasmide pFN476 (ATCC86962) à petit nombre de copies avec sélection par LacZ, par exemple.

Le vecteur pFN476 est coupé au niveau du site Sma I (extrémité franche), purifié, remis en suspension dans 20 µl de ddH₂O et mélangé au produit de PCR du prophage λ en un rapport de 1:3. Après ligation, l'ADN est purifié, remis en suspension dans ddH₂O et transformé dans des cellules DK8-p12C-p37K-p38A « électrocompétentes ». Après rétablissement, les cellules sont étalées sur des plaques de milieu LB X-gal + Chl + Kan + Amp (voir ci-dessus) et mises en incubation à 30 °C.

Quelques colonies blanches sont sélectionnées pour la vérification par PCR de la présence du prophage λ. Une colonie positive de prophages λ est ensuite cultivée pendant une nuit à 30 °C dans du milieu LB + Chl + Kan + Amp pour préparer une culture concentrée de cellules (DK8-T4Mut-λ) à utiliser dans les manipulations ultérieures.

A cette étape, les hôtes transformés sont inductibles par λ à température élevée, lacZ-positifs et contiennent des copies de gènes gp12, gp37 et gp38 de T4 mutés prêts pour la recombinaison homologue.

### Production de la descendance du bactériophage T4 avec des gammes d'hôtes étendues

Une culture fraîche d'une nuit de cellules DK8-T4Mut-λ, est préparée dans du milieu LB + Chl + Kan + Amp à 30 °C.

Les cultures pour infection par T4 sont commencées avec un volume égal ou inférieur à 0,05 ml de cellules d'une culture d'une nuit pour 10 ml de milieu LB + Chl + Kan + Amp afin de garantir que les cellules passent à la phase de croissance exponentielle avant l'addition du bactériophage.

Pour améliorer l'aération, ces cultures sont multipliées dans des fioles d'Erlenmeyer de 250 ml à branche latérale, bouchées non hermétiquement, dans un bain d'eau d'agitateur par secousses à 30 °C.

250 ml de cellules dans du milieu LB + Chl + Kan + Amp sont cultivés à une densité de 3 × 10⁸ cellules par ml à 30 °C avec agitation par secousses.

Des portions aliquotes de 10 ml de cellules à croissance exponentielle sont ensuite transférées à 40 ml de milieu LB + Chl + Kan + Amp préchauffé à 42 °C et mises en incubation pendant exactement 15 min à 42 °C avec aération constante. Du tryptophane est ajouté à une concentration de 0,02 mg/ml et suivi par le bactériophage T4 à une multiplicité d'environ 10 particules par cellule. Les cultures sont transférées à un bain d'eau à 30 °C, en laissant la croissance se poursuivre pendant exactement 25 min.

Le but dans ce cas consiste à isoler la descendance de la première salve et à interrompre la propagation avant que cette descendance de bactériophages de première génération ait eu le temps de se transformer en reproducteurs.

### Récupération de la descendance des bactériophages

Les cellules sont recueillies par centrifugation à 5000 tr/min pendant 5 minutes et le surnageant est récupéré, quelques gouttes de chloroforme sont ajoutées et le mélange est centrifugé de nouveau pendant 10 min à 6000 tr/min. Le surnageant, en excluant le chloroforme, est ajusté à une concentration en tampon SM 1× (MgSO₄ 10 mM, NaCl 100 mM, 0,01 % de gélatine et Tris-HCl 50 mM [pH 7,5]) en utilisant du tampon concentré 5x, et est stocké à 4 °C avant l'analyse.

Les cellules rassemblées en un culot sont remises en suspension dans 8 ml de solution de Tris-chlorhydrate 0,05 M à pH 8,0 avec 25 % de saccharose. Une portion aliquote de 1,6 ml de lysozyme (5 mg/ml) est ajoutée et le mélange est mis en incubation pendant 5 min à 0 °C. Une portion aliquote de 3,2 ml de solution d'EDTA 0,2 M est ajoutée et le mélange est mis en incubation pendant une période de temps supplémentaire de 15 min à 0 °C. Le lysat cellulaire est ajusté à 500 mM de Tris-HCl à pH 7,4, 100 mM de MnCl₂ au moyen de tampon concentré 10×, mis à l'équilibre à 15 °C et mis en incubation avec 10 U de Dnase1 (Sigma) pendant 2 h. Le mélange est ensuite centrifugé à 6000 tr/min pendant 10 min. Le surnageant limpide est prélevé avec précaution, ajusté à une concentration en tampon SM 1 x et stocké de la manière précitée.

### Vérification des extensions des gammes d'hôtes

Des cultures de souches bactériennes pathogènes (*Yersinia* sp., *Salmonella* sp., *E. coli* 0157 H7, *Enterobacter sakazakii,* etc.) sont préparées.

Des portions aliquotes de 3 ml sont ensuite infectées avec 1 ml de culture concentrée de la descendance et mises en incubation à 30 °C avec agitation. La turbidité de la culture est déterminée par colorimétrie immédiatement après l'infection et ensuite toutes les 60 min. Une chute significative de la turbidité de culture en un temps de 5 heures indique que les particules capables d'infecter l'hôte testé étaient présentes dans la descendance de T4 recombinante. Quelques gouttes de chloroforme sont ajoutées à la ou les cultures, ce qui provoque une chute nette de turbidité, et les cultures sont ensuite centrifugées à 5000 tr/min pendant 5 min.

Le surnageant est récupéré et utilisé comme substance infectieuse pour la production de particules de bactériophages dédiées à la bactérie testée, laquelle dans la nature, ne peut être attaquée par le bactériophage sauvage de type T4.

## Revendications

1. Procédé de préparation d'une banque de bactériophages génétiquement modifiés, **caractérisé en ce qu'**il comprend les étapes suivantes:
i) on forme un ensemble de constructions différentes d'ADN, chaque construction étant constituée d'un vecteur de recombinaison homologue et d'au moins un oligonucléotide dont la séquence est produite de manière aléatoire ou un insert correspondant à la copie de tout ou partie d'un gène codant une protéine de ciblage dudit bactériophage dont certaines séquences ont été mutées de manière aléatoire, ledit oligonucléotide ou insert étant destiné à être inséré respectivement dans au moins un gène codant une protéine de ciblage d'un bactériophage, ou dans le gène codant ladite protéine de ciblage d'un bactériophage ;
ii) on transforme des bactéries hôtes à l'aide de cet ensemble de constructions d'ADN;
iii) on infecte les bactéries hôtes transformées à l'étape i) avec ledit bactériophage;
iv) on place les bactéries infectées à l'étape ii) dans des conditions telles que le bactériophage puisse se répliquer, et qu'au moins une partie desdits oligonucléotides différents puisse s'insérer dans au moins un gène codant une protéine de ciblage de ce bactériophage;
v) on récolte les bactériophages recombinants qui se sont répliqués dans lesdites bactéries hôtes Infectées, au terme d'un seul cycle de réplication, en moyenne.

2. Procédé selon la revendication 1, **caractérisé en ce que** l'insertion des oligonucléotides est effectuée par recombinaison homologue.

3. Procédé selon la revendication 2, **caractérisé en ce que** la bactérie hôte est la souche DK8 *d'E.coli*.

4. Procédé selon l'une des revendications 1 à 3, **caractérisé en ce qu'**on récolte les bactériophages à l'étape v), au terme d'un seul cycle, en moyenne, de réplication des bactériophages dans les bactéries hôtes transformées, et avant qu'un second cycle de réplication ne se produise.

5. Procédé selon l'une quelconque des revendications 1 à 4, **caractérisé en ce que** le bactériophage utilisé pour infecter les bactéries hôtes transformées est un bactériophage de la famille T, de préférence un bactériophage de type T4.

6. Procédé selon l'une quelconque des revendications 1 à 5, **caractérisé en ce que** le bactériophage utilisé pour infecter les bactéries hôtes transformées est un bactériophage lui-même modifié, rendu fluorescent par l'introduction d'un gène codant une protéine luminescente ou fluorescente.

7. Procédé selon la revendication 6, **caractérisé en ce que** le bactériophage utilisé pour infecter les bactéries hôtes transformées est un bactériophage présentant une protéine de capside fusionnée à une protéine luminescente ou fluorescente.

8. Procédé selon la revendication 7, **caractérisé en ce que** la protéine luminescente ou fluorescente est fusionnée avec la protéine Hoc de la capside du bactériophage ou bien une partie de celle-ci.

9. Procédé selon l'une quelconque des revendications 1 à 8, **caractérisé en ce que** la protéine de ciblage du bactériophage dont le gène est modifié par insertion d'un oligonucléotide dont la séquence est aléatoire, est choisie parmi les protéines GP12, GP36, GP37 et GP38 ou bien des protéines homologues de celles-ci.

10. Procédé selon l'une quelconque des revendications 1 à 8, **caractérisé en ce que** la protéine de ciblage du bactériophage dont le gène est modifié par insertion d'un oligonucléotide dont la séquence est aléatoire, est la protéine GP12.

## Claims

1. Method for the preparation of a bank of genetically modified bacteriophages, **characterized in that** it comprises the following steps:
i) forming a set of different DNA constructs, each construct being constituted by a homologous recombination vector and at least one oligonucleotide whose sequence is randomly produced or an insert corresponding to the copy of all or part of a gene encoding a targeting protein of said bacteriophage certain sequences of which have been randomly mutated, said oligonucleotide or insert being intended for insertion respectively into at least one gene encoding a targeting protein of a bacteriophage or into the gene encoding said targeting protein of a bacteriophage;
ii) transforming host bacteria by means of this set of DNA constructs;
iii) infecting the host bacteria transformed in step i) with said bacteriophage;
iv) placing the bacteria infected in step ii) in conditions such that the bacteriophage can replicate, and such that at least a part of said different oligonucleotides can be inserted into at least one gene encoding a targeting protein of this bacteriophage;
v) harvesting the recombinant bacteriophages that replicated in said infected host bacteria, on average, after of a single cycle of replication.

2. Method according to claim 1, **characterized in that** insertion of the oligonucleotides is carried out by homologous recombination.

3. Method according to claim 2, **characterized in that** the host bacterium is the DK8 strain of *E*. *coli.*

4. Method according to one of claims 1 to 3, **characterized in that** the bacteriophages are harvested in step v), on average, after a single cycle of replication of the bacteriophages in the transformed host bacteria, before a second replication cycle takes place.

5. Method according to any one of claims 1 to 4, **characterized in that** the bacteriophage used for infecting the transformed host bacteria is a bacteriophage of the T family, preferably a bacteriophage of T4 type.

6. Method according to any one of claims 1 to 5, **characterized in that** the bacteriophage used for infecting the transformed host bacteria is a bacteriophage itself modified and made fluorescent by the introduction of a gene encoding a luminescent or fluorescent protein.

7. Method according to claim 6, **characterized in that** the bacteriophage used for infecting the transformed host bacteria is a bacteriophage having a capsid protein fused with a luminescent or fluorescent protein.

8. Method according to claim 7, **characterized in that** the luminescent or fluorescent protein is fused with the Hoc protein of the capsid of the bacteriophage or a part thereof.

9. Method according to any one of claims 1 to 8, **characterized in that** the targeting protein of the bacteriophage whose gene is modified by insertion of an oligonucleotide whose sequence is random, is selected from the proteins GP12, GP36, GP37 and GP38 or proteins that are homologues of the latter.

10. Method according to any one of claims 1 to 8, **characterized in that** the targeting protein of the bacteriophage whose gene is modified by insertion of an oligonucleotide whose sequence is random, is the protein GP12.

## Patentansprüche

1. Verfahren zur Herstellung einer Bank von genetisch modifizierten Bakteriophagen, **dadurch gekennzeichnet, dass** es die folgenden Schritte umfasst:
i) man bildet eine Gesamtheit von unterschiedlichen DNA-Konstrukten, wobei jedes Konstrukt gebildet wird aus einem homologen Rekombinationsvektor und mindestens einem Oligonukleotid, dessen Sequenz auf zufällige Weise erzeugt wird, oder einem Insert, welches der Kopie der Gesamtheit oder eines Teils eines Gens, welches ein Targeting-Protein des Bakteriophagen kodiert, von welchem bestimmte Sequenzen auf zufällige Weise mutiert worden sind, entspricht, wobei das Oligonukleotid bzw. Insert dazu bestimmt ist, in mindestens ein Gen, welches ein Targeting-Protein eines Bakteriophagen kodiert, bzw. in das Gen, welches das Targeting-Protein eines Bakteriophagen kodiert, inseriert zu werden;
ii) man transformiert Wirtsbakterien mit Hilfe dieser Gesamtheit von DNA-Konstrukten;
iii) man infiziert die in Schritt i) transformierten Wirtsbakterien mit dem Bakteriophagen;
iv) man setzt die in Schritt ii) infizierten Bakterien solchen Bedingungen aus, dass der Bakteriophage sich replizieren kann und dass mindestens ein Teil der unterschiedlichen Oligonukleotide sich in mindestens ein Gen, welches ein Targeting-Protein von diesem Bakteriophagen kodiert, inserieren kann;
v) man gewinnt die rekombinanten Bakteriophagen, die sich in den infizierten Wirtsbakterien repliziert haben, im Mittel am Ende eines einzigen Replikationszyklus.

2. Verfahren nach Anspruch 1, **dadurch gekennzeichnet, dass** die Insertion der Oligonukleotide durch homologe Rekombination erfolgt.

3. Verfahren nach Anspruch 2, **dadurch gekennzeichnet, dass** das Wirtsbakterium der Stamm DK8 von *E*. *coli* ist.

4. Verfahren nach einem der Ansprüche 1 bis 3, **dadurch gekennzeichnet, dass** man die Bakterien in dem Schritt v) im Mittel am Ende eines einzigen Replikationszyklus der Bakteriophagen in den transformierten Wirtsbakterien und bevor ein zweiter Replikationszyklus erfolgt, gewinnt.

5. Verfahren nach einem der Ansprüche 1 bis 4, **dadurch gekennzeichnet, dass** der Bakteriophage, der eingesetzt wird, um die transformierten Wirtsbakterien zu infizieren, ein Bakteriophage der Familie T, vorzugsweise ein Bakteriophage vom Typ T4 ist.

6. Verfahren nach einem der Ansprüche 1 bis 5, **dadurch gekennzeichnet, dass** der Bakteriophage, der eingesetzt wird, um die transformierten Wirtsbakterien zu infizieren, ein seinerseits modifizierter Bakteriophage ist, der durch die Einführung eines Gens, welches ein lumineszierendes oder fluoreszierendes Protein kodiert, fluoreszierend gemacht worden ist.

7. Verfahren nach Anspruch 6, **dadurch gekennzeichnet, dass** der Bakteriophage, der eingesetzt wird, um die transformierten Wirtsbakterien zu infizieren, ein Bakteriophage ist, welcher ein an ein lumineszierendes oder fluoreszierendes Protein fusioniertes Kapsidprotein aufweist.

8. Verfahren nach Anspruch 7, **dadurch gekennzeichnet, dass** das lumineszierende oder fluoreszierende Protein mit dem Protein Hoc des Kapsids des Bakteriophagen oder alternativ einem Teil von jenem fusioniert ist.

9. Verfahren nach einem der Ansprüche 1 bis 8, **dadurch gekennzeichnet, dass** das Targeting-Protein des Bakteriophagen, dessen Gen durch Insertion eines Oligonukleotids, dessen Sequenz zufällig ist, modifiziert wird, unter den Proteinen GP12, GP36, GP37 und GP38 oder alternativ zu jenen homologen Proteinen ausgewählt ist.

10. Verfahren nach einem der Ansprüche 1 bis 8, **dadurch gekennzeichnet, dass** das Targeting-Protein des Bakteriophagen, dessen Gen durch Insertion eines Oligonukleotids, dessen Sequenz zufällig ist, modifiziert wird, das Protein GP12 ist.
